# EUROPÄISCHE PATENTSCHRIFT

(11) **EP 1 525 176 B1**
(45) Veröffentlichungstag und Bekanntmachung des Hinweises auf die Patenterteilung: **31.12.2008**
(21) Anmeldenummer: 03764961.3
(22) Anmeldetag: 10.07.2003
(51) Int. Cl.: C07C 45/48, C07C 49/385, C07C 49/587

(54) **VERFAHREN ZUR HERSTELLUNG VON MAKROCYCLISCHEN KETONEN DURCH DIECKMANN-KONDENSATION IN DER GASPHASE**
METHOD FOR PRODUCING MACROCYCLIC KETONES BY MEANS OF DIECKMANN CONDENSATION IN THE GAS PHASE
PROCEDE DE PRODUCTION DE CETONES MACROCYCLIQUES PAR CONDENSATION DE DIECKMANN EN PHASE GAZEUSE

(30) Priorität: 18.07.2002 DE 10232750
(43) Veröffentlichungstag der Anmeldung: 27.04.2005
(73) Patentinhaber: BASF SE, 67056 Ludwigshafen (DE)
(72) Erfinder: WARTINI, Alexander, 69120 Heidelberg (DE); EBEL, Klaus, 68623 Lampertheim (DE); HIEBER, Gisela, 69120 Heidelberg (DE); WEIGL, Hagen, 68526 Ladenburg (DE)
(86) Internationale Anmeldenummer: PCT/EP2003/007455
(87) Internationale Veröffentlichungsnummer: WO 2004/009524

(56) Entgegenhaltungen:
- EP-A- 0 251 111
- EP-A- 0 352 674
- EP-A- 0 400 509
- US-A- 1 702 842
- US-A- 1 702 843
- US-A- 4 335 261

## Beschreibung

Die vorliegende Erfindung betrifft ein Verfahren zur Herstellung von macrocyclischen Ketonen (C₁₃-C₂₀) durch Dieckmann-Kondensation in der Gasphase an einem heterogenen Katalysator. Das Verfahren findet insbesondere Anwendung bei der Herstellung von Riechstoffen, beispielsweise Zibeton und Exalton, die in breiten Bereichen der Parfüm- und Kosmetikindustrie eingesetzt werden.

Die bisher angewandten Synthesemethoden basieren zumeist auf einer konventionellen intramolekularen Kondensationsreaktion, wie der Dieckmann-Kondensation in der Flüssigphase.

Die Synthese beispielsweise von Zibeton durch Dieckmann-Cyclisierung von 9-Octadecen-1,18-dicarbonsäuredialkylestern und anschließende verseifung und Decarboxylierung des entsprechen erhaltenen β-Ketoesters ist schon lange bekannt (z.B.: J. Tsuji, Tetrahedron Lett., 21, 2955-2958 (1980) ; Y. Choo, J. Am. Oil Chem. Soc. 71, 911-913 (1994))

Von H.W. Bost (Perfumer & Flavorist, 1982, 7, 57) wird eine Reaktion eines gesättigten Diesters in der Gasphase an einem Thoriumoxid-Katalysator beschrieben. Die Ausbeuten liegen hier aber mit 14 % deutlich niedriger als in den gängigen Cyclisierungen unter Hochverdünnung.

Die EP 0 251 111 beschreibt ein Verfahren zur Herstellung cyclischer Ketone mit 5 bis 7 Kohlenstoffatomen im Ring durch Umsetzung der entsprechenden Dicarbonsäureester bei Temperaturen von 150 bis 450°C an festen oxidischen Katalysatoren.

Die bislang bekannten Methoden zur Herstellung von macrozyclischen Ketonen aus linear endständigen Diestern haben jedoch folgende gravierende Nachteile:
1) Es muss eine starke Base in stöchiometrischer Menge eingesetzt werden.
2) Die Reaktion muss zur Erzielung guter Ausbeuten unter hoher Verdünnung durchgeführt werden.
3) Der intermediär gebildete β-Ketoester muss in einem getrennten Schritt verseift und decarboxyliert werden.
4) Bei der Gasphasenreaktion werden extrem niedrige Ausbeuten erzielt. Der verwendete Katalysator ist teuer.

Aufgabe der vorliegenden Erfindung war es ein Herstellverfahren für makrocyclische Ketone zu entwickeln, dass eine vereinfachte, wirtschaftlichere Herstellweise ermöglicht.

Die Aufgabe wurde erfindungsgemäß gelöst, durch ein Verfahren zur Herstellung von makrocyclischen Ketonen der allgemeinen Formel I 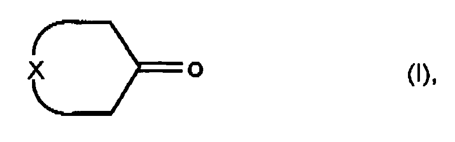 wobei
- X: einen ein- oder mehrfach ungesättigten oder gesättigten C₁₀-C₁₇-Alkylrest bedeutet,
der gegebenenfalls durch einen C₁-C₆-Alkylrest substituiert sein kann,
durch direkte Cyclisierung von Verbindungen der allgemeinen Formel II 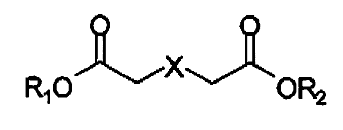 wobei
- R₁, R₂: jeweils unabhängig voneinander gleich oder verschieden sein können und Wasserstoff oder C₁-C₆-Alkyl bedeuten und X die oben angegebene Bedeutung besitzt, in der Gasphase an einem heterogenen Festbettkatalysator, enthaltend als Aktivkomponenten Oxide, Hydroxide oder Carboxylate der IV. Nebengruppe, der mit Oxiden der I. Hauptgruppe dotiert ist.

Dabei werden die linear endständigen Dicarbonsäuren, Monoester-monocarbonsäuren bzw. die Dialkylester der allgemeinen Formel II verdampft und in der Gasphase an heterogenen Katalysatoren direkt zum Keton cyclisiert.

Unter einem ein- oder mehrfach ungesättigten oder gesättigten C₁₀-C₁₇-Alkylrest versteht man beispielsweise einen
-(CH₂)₁₂-
-(CH₂)₁₄-
-CH(CH₃)-(CH₂)₁₁-
-CH=CH-(CH₂)₈-CH=CH-
-CH₂-CH₂-CH=CH-(CH₂)₆-CH=CH-CH₂-CH₂-
- (CH₂)₆-CH=CH-(CH₂)₆- oder einen
-CH₂-CH₂-CH=CH- (CH₂)₈-CH₂-CH=CH-CH₂-Rest,
bevorzugt einen - (CH₂)₁₂- oder einen (CH₂)₆-CH=CH-(CH₂)₆-Rest.

Unter einem C₁-C₆-Alkylrest versteht man, wenn nicht anders angegeben, einen geradkettigen oder verzweigten Alkylrest mit 1 bis 6 Kohlenstoffatomen. Bevorzugt sind die Methyl-, Ethyl-, n-Propyl-, i-Propyl-, n-Butyl-, t-Butyl-, Pentyl- oder die Hexylgruppe.

In einer bevorzugten Ausführungsform werden durch das erfindungsgemäße Verfahren die Riechstoffe Exalton (Cyclopentadecanon) oder Cibeton (cis-9-Cycloheptadecen-1-on) hergestellt.

Die Herstellung der Verbindungen der Formel II erfolgt nach an sich bekannten Methoden, wie sie in der Literatur (z.B. JAOCS, Vol. 71, No. 8 (1994), S. 911-913 oder Tetrahedron Lett. Vol. 21, (1980), S. 2955-2958)) beschrieben sind, und zwar unter Reaktionsbedingungen, wie sie für die Synthese bekannt und geeignet sind. Dabei kann man auch von an sich bekannten, hier nicht näher erwähnten Varianten Gebrauch machen.

Die Ausgangsstoffe der Verbindungen der Formel II können gewünschtenfalls auch in situ, ohne vorherige Isolierung der Verbindungen der Formel II direkt weiter zu den Verbindungen der Formel I umgesetzt werden.

Der Vorteil des erfindungsgemäßen Verfahrens besteht darin, dass die normalerweise bei einer in Lösung durchgeführten Dieckmann-Kondensation üblichen Schritte Cyclisierung, Verseifung und anschließende Decarboxylierung in einem einzigen Schritt in der Gasphase, ohne Isolierung der jeweiligen Zwischenprodukte stattfinden. Der weitere Vorteil besteht darin, dass weniger Lösungsmittel benötigt wird, was zu einem Kostenvorteil und einer reduzierten Abfallmenge führt, die gegebenenfalls aufgearbeitet werden müsste.

Das Verfahren kann sowohl in der Wirbelschicht als auch im Festbett durchgeführt werden. Vorzugsweise findet die Umsetzung jedoch im Festbett statt.

Die Cyclisierung wird bei Temperaturen von 200 bis 600°C, bevorzugt 250 bis 500°C, durchgeführt. Grundsätzlich ist die Reaktion sowohl im Vakuum, bei Normaldruck und unter erhöhtem Druck möglich. Zur einfacheren Verdampfung der hochsiedenden Edukte ist die Umsetzung im Vakuum oder bei Normaldruck vorzuziehen.

Die Reaktion kann gegebenenfalls unter Zugabe geringer Mengen Wasser durchgeführt werden, um nach der Cyclisierung Esterhydrolyse-Schritte zu begünstigen. Das Wasser kann dabei auf einmal oder in mehreren Schritten an einem beliebigen Punkt des Verfahrens, jedoch bevor das Gemisch den Katalysator vollständig passiert hat, zugegeben werden (z.B. enthält II bereits Wasser oder Wasser wird im Verdampfer zugesetzt).

Geeignete Katalysatoren für das erfindungsgemäße Verfahren sind heterogene Festbettkatalysatoren, welche als Aktivkomponenten Oxide, Hydroxide oder Carboxylate der IV. Nebengruppe enthalten, die in der Lage sind Carbonsäuren, Carbonsäureester oder Carbonsäurenitrile in der Gasphase zu Ketonen umzusetzen. Die Katalysatoren sind noch mit Oxiden der I. Hauptgruppe dotiert und können sowohl als Vollkontakte, als auch als Trägerkatalysatoren eingesetzt werden. Als Trägermaterialien kommen in der Katalysatorchemie übliche Materialien beispielsweise SiO₂ oder Al₂O₃ in Frage.

Mit besonderem Vorteil verwendet man TiO₂, insbesondere mit Alkalimetalloxiden dotiertes TiO₂, d.h. etwa 2 bis 10 Gew.-% Na₂O und/oder K₂O enthaltendes TiO₂. Die Herstellung der Katalysatoren erfolgt gemäß den in EP 352 674 beschriebenen Verfahren.

Zur Durchführung des Verfahrens wird die Verbindung II als Flüssigkeit oder als Schmelze oder als Lösung in einem inerten organischen Lösungsmittel, beispielsweise Toluol oder Tetrahydrofuran verdampft und anschließend gegebenenfalls in Gegenwart eines inerten Gases oder Gemische verschiedener inerter Gase, wie Stickstoff, Kohlendioxid oder Helium bei der gewünschten Reaktionstemperatur gasförmig über den fest angeordneten Katalysator geleitet. Um nach der Cyclisierung Esterhydrolyse-Schritte zu begünstigen, kann die Reaktion unter Zusatz geringer Mengen Wasser durchgeführt werden. Vorteilhaft arbeitet man mit 0 bis 30 Gew. -% Wasser, bevorzugt mit 5 bis 15 Gew. -% Wasser bezogen auf die Menge an eingesetztem II.

Die Reaktionsprodukte werden mittels geeigneter Kühlvorrichtungen kondensiert oder mit einem geeigneten Lösungsmittel niedergeschlagen (Quench) und anschließend fraktioniert destilliert.
Reaktionsprodukte mit noch unumgesetztem Ausgangsmaterial lassen sich gegebenenfalls erneut ohne weitere Reinigung direkt in die Cyclisierungsreaktion zurückführen.

Das Verfahren kann sowohl kontinuierlich als auch als Batch-Verfahren durchgeführt werden. Bevorzugt ist jedoch die kontinuierliche Fahrweise.

Das erfindungsgemäße Verfahren benötigt keine stöchiometrische Menge starker Basen keine Lösungsmittel oder sonstige Hilfsstoffe und liefert direkt die makrocyclischen Ketone in guten Ausbeuten und Selektivitäten.

### Beispiele

### Beispiel 1: Herstellung von Exalton aus 1,16-Hexadecandisäurediethylester

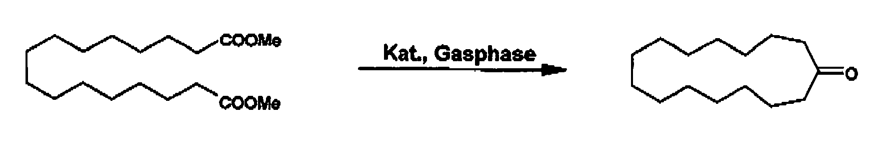

Eine Lösung von 10 g 1,16-Hexadecandisäurediethylester in 120 ml Tetrahydrofuran wurde in einem Gasphasen-Rohrreaktor bei 270°C verdampft und mit Stickstoff bei 350°C über einen Katalysator (TiO₂ + 2 % K₂O) geleitet. Anschließend wurde kondensiert und fraktioniert destilliert. Die Ausbeute betrug 78 % bei einer Selektivität von > 90 %.

### Beispiel 2: Herstellung von Zibeton aus 1,18-Octadec-9-endicarbonsäuredimethylester

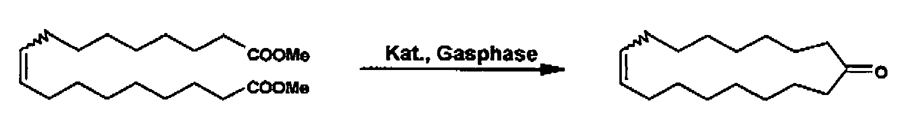

Eine Lösung von 1,18-Octadec-9-endicarbonsäuredimethylester in Toluol in einem Volumenverhältnis von 1:9 wurde soweit mit Wasser gesättigt, dass es gerade nicht mehr zu einer Phasentrennung kommt. Diese Lösung wurde bei 380°C in einem Gasphasen-Rohrreaktor verdampft und mit Stickstoff bei 450°C über einen Katalysator (TiO₂ + 2 % K₂O) geleitet. Anschließend wurde kondensiert und fraktioniert destilliert. Die Ausbeute betrug 45 % bei einer Selektivität von 70 %.

## Patentansprüche

1. Verfahren zur Herstellung von makrocyclischen Ketonen der allgemeinen Formel I 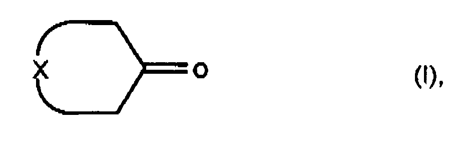 wobei
X einen ein- oder mehrfach ungesättigten oder gesättigten C₁₀-C₁₇-Alkylrest bedeutet, der gegebenenfalls durch einen C₁-C₆-Alkylrest substituiert sein kann,
durch direkte Cyclisierung von Verbindungen der allgemeinen Formel II 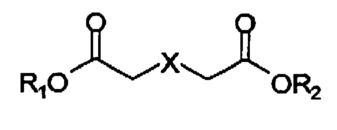 wobei
R₁, R₂ jeweils unabhängig voneinander gleich oder verschieden sein können und C₁-C₆-Alkyl bedeuten und X die oben angegebene Bedeutung besitzt, in der Gasphase an einem heterogenen Festbettkatalysator, enthaltend als Aktivkomponenten Oxide, Hydroxide oder Carboxylate der IV. Nebengruppe, der mit Oxiden der I. Hauptgruppe dotiert ist.

2. Verfahren gemäß Anspruch 1, **dadurch gekennzeichnet, dass** die Umsetzung bei Temperaturen von 200 bis 600°C stattfindet.

3. Verfahren gemäß einem der Ansprüche 1 oder 2, **dadurch gekennzeichnet, dass** als Katalysator TiO₂ verwendet wird.

4. Verfahrengemäßeinem der Ansprüche 1 bis 3, **dadurch gekennzeichnet, dass** als Katalysator mit Alkalimetalloxiden dotiertes TiO₂ verwendet wird.

5. Verfahren gemäß einem der Ansprüche 1 bis 4, **dadurch gekennzeichnet, dass** die Verbindungen der Formel I, ausgewählt sind aus der Gruppe Exalton oder Cibeton.

6. Verfahren gemäß einemderAnsprüche 1 bis 5, **dadurch gekennzeichnet, dass** die Verbindungen der Formel II ausgewählt sind aus der Gruppe 1,16-Hexadecandisäuredimethylester oder 1,18-Octadec-9-endicarbonsäuredimethylester.

7. Verfahren gemäß einem der Ansprüche 1 bis 6, **dadurch gekennzeichnet, dass** die Reaktion in Gegenwart von 0 bis 30 Gew.-% Wasser bezogen auf die eingesetzte Verbindung der Formel II durchgeführt wird.

## Claims

1. A process for the preparation of macrocyclic ketones of the formula I 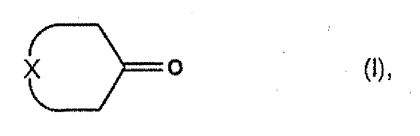 where
X is a mono- or polyunsaturated or saturated C₁₀-C₁₇-alkyl radical which may optionally be substituted by a C₁-C₆-alkyl radical,
by direct cyclization of compounds of the formula II 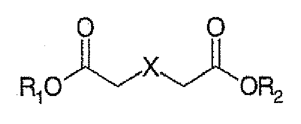 where
R₁, R₂, in each case independently of the other, may be identical or different and are C₁-C₆-alkyl and X has the meaning given above, in the gas phase over a heterogeneous fixed-bed catalyst comprising, as active components, oxides, hydroxides or carboxylates of subgroup IV,
which catalyst is doped with oxides of main group I.

2. The process according to claim 1, wherein the reaction takes place at temperatures of from 200 to 600°C.

3. The process according to either claim 1 or 2, wherein the catalyst used is TiO₂.

4. The process according to any of claims 1 to 3, wherein the catalyst used is TiO₂ doped with alkali metal oxides.

5. The process according to any of claims 1 to 4, wherein the compounds of the formula I are chosen from the group consisting of exaltone or civetone.

6. The process according to any of claims 1 to 5, wherein the compounds of the formula II are chosen from the group consisting of dimethyl 1,16-hexadecanedioate or dimethyl 1,18-octadec-9-enedicarboxylate.

7. The process according to any of claims 1 to 6, wherein the reaction is carried out in the presence of from 0 to 30% by weight of water, based on the compound of the formula II used.

## Revendications

1. Procédé pour la préparation de cétones macrocycliques de formule générale I, 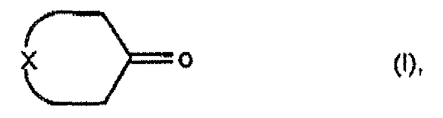 où
X signifie un radical alkyle en C₁₀-C₁₇ monoinsaturé, polyinsaturé ou saturé qui peut le cas échéant être substitué par un radical alkyle en C₁-C₆,
par cyclisation directe de composés de formule générale II 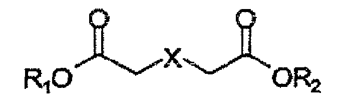 où
R₁, R₂ peuvent être à chaque fois, indépendamment l'un de l'autre, identiques ou différents et signifient alkyle en C₁-C₆ et X a la signification susmentionnée, en phase gazeuse sur un catalyseur en lit fixe hétérogène, contenant comme composants actifs des oxydes, des hydroxydes ou des carboxylates du IVème groupe secondaire, qui est dopé avec des oxydes du Ier groupe principal.

2. Procédé selon la revendication 1, **caractérisé en ce que** la transformation a lieu à des températures de 200 à 600°C.

3. Procédé selon l'une quelconque des revendications 1 ou 2, **caractérisé en ce qu'**on utilise comme catalyseur du TiO₂.

4. Procédé selon l'une quelconque des revendications 1 à 3, **caractérisé en ce qu'**on utilise comme catalyseur du TiO₂ dopé avec des oxydes de métal alcalin.

5. Procédé selon l'une quelconque des revendications 1 à 4, **caractérisé en ce que** les composés de formule I sont choisis dans le groupe formé par l'Exalton ou le Cibeton.

6. Procédé selon l'une quelconque des revendications 1 à 5, **caractérisé en ce que** les composés de formule II sont choisis dans le groupe formé par l'ester diméthylique de l'acide 1,16-hexadécanedioïque ou l'ester diméthylique de l'acide 1,18-octadéc-9-ènedicarboxylique.

7. Procédé selon l'une quelconque des revendications 1 à 6, **caractérisé en ce que** la réaction est réalisée en présence de 0 à 30% en poids d'eau par rapport au composé utilisé de formule II.
